# EUROPÄISCHE PATENTSCHRIFT

(11) **EP 2 621 421 B1**
(45) Veröffentlichungstag und Bekanntmachung des Hinweises auf die Patenterteilung: **04.03.2015**
(21) Anmeldenummer: 10765955.9
(22) Anmeldetag: 30.09.2010
(51) Int. Cl.: A61F 9/007, A61F 9/009

(54) **EINRICHTUNG FÜR DIE AUGENCHIRURGIE**
DEVICE FOR EYE SURGERY
DISPOSITIF DE CHIRURGIE OCULAIRE

(43) Veröffentlichungstag der Anmeldung: 07.08.2013
(73) Patentinhaber: WaveLight GmbH, 91058 Erlangen (DE)
(72) Erfinder: JEGLORZ, Tobias, 90547 Stein (DE); DONITZKY, Christof, 90542 Eckental/Eschenau (DE)
(74) Vertreter: Katérle, Axel
(86) Internationale Anmeldenummer: PCT/EP2010/005975
(87) Internationale Veröffentlichungsnummer: WO 2012/041350

(56) Entgegenhaltungen:
- EP-A2- 0 836 868
- WO-A1-02/087451
- WO-A1-2007/006433
- US-A1- 2003 144 678
- US-B1- 6 506 198

## Beschreibung

Die Erfindung betrifft eine Einrichtung für die Augenchirurgie, umfassend eine Vakuumpumpanordnung zur Erzeugung eines zur Fixierung eines Saugrings an einem Auge dienenden Vakuums, ein Evakuierungswegesystem zur Übertragung des Vakuums zu einem Schnittstellenanschluss, welcher den lösbaren Anschluss eines den Saugring umfassenden Saugringinstrumentariums gestattet, sowie eine Steuereinheit zur Steuerung der Vakuumpumpanordnung.

Eine derartige Einrichtung kann beispielsweise im Rahmen eines Mikrokeratoms zum Einsatz kommen, d.h. bei einem mikrochirurgischen Hobel, wie er beispielsweise für die Anbringung eines Flap-Schnitts an einem humanen Auge verwendet wird. Ein solcher Flap-Schnitt ist nötig im Rahmen einer in der Fachwelt üblicherweise als LASIK (Laser In-Situ Keratomileusis) bekannten Operationsform. Mittels des Flap-Schnitts wird dabei an der Oberfläche der Kornea ein Scheibchen (Flap) herausgeschnitten, das an einem Teil seines Umfangs noch mit dem übrigen Korneagewebe verbunden ist und deshalb problemlos zur Seite geklappt und anschließend wieder zurückgeklappt werden kann. Je nachdem, ob der Flap-Schnitt im Stroma oder im Epithel an der Bowman-Membran verläuft, spricht man in der Fachwelt üblicherweise von klassischer LASIK bzw. Epi-LASIK.

Der Saugring wird bei einem Mikrokeratom nicht nur zur Fixation des Auges benötigt sondern üblicherweise auch zur Ankopplung und Führung einer mit einer Schneidklinge bestückten Schneideinheit, die in der Regel vom Operateur in der Hand gehalten wird und deshalb häufig auch als Handstück des Mikrokeratoms bezeichnet wird. Teil des Handstücks ist üblicherweise auch eine motorische Antriebseinheit, welche der Erzeugung der Schneidbewegung der Schneidklinge dient. Bei einem von der Firma WaveLight unter der Handelsbezeichnung RONDO vertriebenen Mikrokeratom besitzt die Schneidklinge ähnlich einem Rasiermesser eine geradlinige Schneidkante und wird unter hochfrequenter lateraler Oszillation linear in Richtung senkrecht zur Schneidkante bewegt. Die Schneidklinge ist beispielsweise in einem Schneidkopf gehalten, welcher lösbar und somit auswechselbar mit einem Antriebsmodul des Handstücks gekoppelt ist.

Das Saugringinstrumentarium kann neben dem eigentlichen Saugring weitere Komponenten umfassen, die z.B. der Weiterleitung des Vakuums von dem Schnittstellenanschluss zu dem Saugring dienen. Beispielsweise können diese Komponenten eine Schlauchleitung mit einem geeigneten Verbinder zur Verbindung mit dem Schnittstellenanschluss umfassen. Am schnittstellenfernen Ende kann diese Schlauchleitung mit einem Anschlussstutzen einer den Saugring bildenden, beispielsweise einstückig hergestellten Saugringeinheit lösbar verbunden sein. Es ist darauf hinzuweisen, dass die Saugringeinheit durchaus mehrere Schlauchleitungsanschlüsse aufweisen kann, die den Anschluss mehrerer Schlauchleitungen gestatten, insbesondere wenn die Saugringeinheit mehrere getrennt evakuierbare Evakuierungsräume (Saugkammern) aufweist. Zur Fixierung des Saugrings am Auge weist dieser üblicherweise eine ausschließlich zwischen dem Saugring und der Augenoberfläche begrenzte Ringkammer auf.

Aus Dokument US 2003/0144678 A1 ist eine chirurgische Vorrichtung zum Schneiden der Kornea eines Patentenauges und zur Erzeugung eines aus kornealem Gewebe bestehenden Flaps bekannt. Dabei verfügt die Vorrichtung über Mittel zum Halten und Positionieren des Auges. Die Halte- und Positionierungsmittel umfassen einen Positionierungsring mit einem Verbindungselement, welches dazu eingerichtet ist, über einen Vakuumschlauch mit einer Vakuumpumpe derart verbunden zu werden, dass bei einem Ansaugen der Positionierungsring über einem Abschnitt der Kornea des Auges gehalten wird. Die Vakuumpumpe wird anhand einer Prozessorsteuerung gesteuert. Wenn die Prozessorsteuerung eingeschalten wird, werden einen Anzahl interner Test durchgeführt und die Vakuumpumpe erzeugt zunächst eine Vakuum in einem Reservevakuumtank um im Anschluss daran so lange weiterzulaufen, bis ein gewünschtes Vakuum relativ zu einem Atmosphärendruck erzeugt ist.

Eine erfindungsgemäße Einrichtung kann alternativ oder zusätzlich im Rahmen einer lasertechnischen Schneidbearbeitung des menschlichen Auges zum Einsatz kommen. Diesbezügliche Lasersysteme sehen oftmals die Zwischenschaltung eines Adapters zwischen dem zu bearbeitenden Auge und einem die Laserstrahlung auf das Auge fokussierenden Objektiv vor. Der Adapter bewirkt eine Positionierung des Auges gegenüber dem Lasersystem, was die zielgenaue Schnitterzeugung erleichtert. Dabei ist es bekannt, den Adapter mit einem für die Laserstrahlung transparenten Kontaktelement auszuführen, welches in flächigen Kontakt mit dem Auge gebracht wird. Im Fall einer planen Kontaktfläche des Kontaktelements erfolgt eine Einebnung der Kornea; man spricht deshalb von einem Applanationselement.

Durch gegenseitige Kopplung des seinerseits am Auge festgesaugten Saugrings und des Adapters, etwa durch Ansaugen des Adapters an den Saugring, ist es möglich, das Auge präzise und sicher gegenüber dem Lasersystem zu positionieren.

Ungeachtet der Operationsform, bei der das Saugringinstrumentarium eingesetzt wird, ist ein ausreichend starkes Vakuum nötig, damit der Saugring während der Operation zuverlässig am Auge festgesaugt bleibt. Die Vakuumpumpanordnung ist in der Regel ausreichend leistungsstark dimensioniert, um unter Normalbedingungen das benötigte Vakuum aufbauen zu können. Eine Überdimensionierung der Vakuumpumpanordnung ist freilich aus Kosten- und Bauraumgründen in der Regel nicht erwünscht.

Die erzielbare Qualität des Vakuums hängt nicht nur von der maximalen Pumpleistung der Vakuumpumpanordnung ab sondern auch von der Höhenlage des Einsatzortes und von der aktuellen Wettersituation. Bei gegebener Pumpleistung hängt der erzielbare Vakuumdruck vom aktuellen Atmosphärendruck am Einsatzort ab, wobei dieser Atmosphärendruck je nach Höhenlage des Einsatzorts und je nach Wetterlage (Hochdruck, Tiefdruck) mehr oder weniger stark schwanken kann. Dies kann dazu führen, dass bei einem in großer Höhe liegenden Einsatzort unter Umständen nur ein erheblich kleinerer Vakuum-Arbeitsbereich verfügbar ist als unter den Bedingungen des Flachlands. Selbst wenn der Anwender an einer Bedienkonsole einen bestimmten Sollwert für den relativen Unterdruck vorgibt (relativer Unterdruck meint die Höhe des Unterdrucks gegenüber der Umgebung), kann der tatsächlich erzielte relative Unterdruck substantiell kleiner sein (z.B. -500 mmHg statt eines benutzerseitig gewünschten Werts von z.B. -600 mmHg) und daher unter Umständen operative Gefahren hervorrufen.

Aufgabe der Erfindung ist es, eine augenchirurgische Einrichtung der eingangs bezeichneten Art derart weiterzubilden, dass höhen- oder wetterbedingte Einschränkungen des Vakuum-Arbeitsbereichs der Vakuumpumpanordnung erkennbar sind.

Diese Aufgabe wird durch eine Einrichtung für die Augenchirurgie gemäß Anspruch 1 gelöst.

Zur Lösung dieser Aufgabe schlägt die Erfindung vor, die augenchirurgische Einrichtung mit Druckmesskomponenten zur Messung zumindest des Vakuumdrucks auszurüsten, wobei die Steuereinheit dazu eingerichtet ist, einen Differenzdruck zwischen dem gemessenen Vakuumdruck und einem Atmosphärendruck zu ermitteln. Vorteilhafterweise sind die Druckmesskomponenten dabei auch zur Messung des Atmosphärendrucks ausgebildet, so dass die Steuereinheit den Differenzdruck aus dem gemessenen Vakuumdruck und dem gemessenen Atmosphärendruck ermitteln kann. Alternativ ist eine Eingabe durch einen Benutzer vorstellbar, wobei diese Eingabe für den Atmosphärendruck repräsentativ ist. Beispielsweise ist es vorstellbar, in einem Speicher tabellen- oder formelmäßig Informationen über die Korrespondenz zwischen der Höhenlage über Meeresniveau und einem zugehörigen nominellen Atmosphärendruck abzuspeichern. Der Benutzer kann dann eine Angabe über die Höhenlage des Einsatzortes eingeben, woraus die Steuereinheit unter Zugriff auf die gespeicherten Informationen einen Wert für den Atmosphärendruck ermittelt.

Wenn hier von einer Steuerung der Vakuumpumpanordnung die Rede ist, so versteht es sich, dass darunter eine Regelung im regelungstechnischen Sinn fällt. Der Begriff Steuerung wird hier als Allgemeinbegriff für eine Steuerung ohne Rückkopplung und eine Steuerung mit Rückkopplung (Regelung) verwendet.

Die Steuereinheit ist dazu eingerichtet, einen Testbetriebslauf mindestens einer Vakuumpumpe der Vakuumpumpanordnung mit einer vorbestimmten Einstellung der Pumpleistung zu bewirken und den Differenzdruck auf Grundlage eines oder mehrerer während dieses Testbetriebslaufs gemessener Werte für den Vakuumdruck zu ermitteln. Der Testbetriebslauf kann von der Steuereinheit automatisch jedes Mal dann initiiert werden, wenn sich ein vorbestimmter Auslöser ereignet. Ein solcher Auslöser ist insbesondere das Einschalten einer elektrischen Energieversorgung der Steuereinheit und der Vakuumpumpanordnung. Wird die augenchirurgische Einrichtung beispielsweise im Klinikbetrieb morgens eingeschaltet und abends wieder ausgeschaltet, wird der Testbetriebslauf täglich einmal durchgeführt, und zwar morgens nach dem Einschalten. Es ist selbstverständlich vorstellbar, alternativ oder zusätzlich weitere auslösende Momente vorzugeben, die eine Durchführung des Testbetriebslaufs veranlassen können. Beispielsweise kann der Testbetriebslauf gestartet werden, wenn eine vorbestimmte Zeit lang kein Testbetriebslauf durchgeführt wurde.

Der Testbetriebslauf soll dazu dienen, den tatsächlich erzielten relativen Unterdruck zu ermitteln und daran anknüpfend beurteilen zu können, ob eine ausreichend hohe Saugkraft an dem Saugring erzeugbar ist, um eine Operation zuverlässig durchführen zu können. Hierzu ist es zweckmäßig, wenn die Steuereinheit dazu eingerichtet ist, den Testbetriebslauf ohne an den Schnittstellenanschluss angeschlossenes Saugringinstrumentarium durchzuführen. Insbesondere kann die Steuereinheit dazu eingerichtet sein, den Testbetriebslauf nur dann durchzuführen, wenn das Saugringinstrumentarium nicht an den Schnittstellenanschluss angeschlossen ist. Um ohne angeschlossenes Saugringinstrumentarium einen abgedichteten Raum bereitzustellen, in dem das Vakuum testweise erzeugt werden kann, ist es vorstellbar, den Schnittstellenanschluss mit einer Absperrfunktion auszugestalten, die eine Absperrung des Evakuierungswegesystems nach außen bewirkt, wenn das Saugringinstrumentarium nicht angeschlossen ist. Durch das Anschließen des Saugringinstrumentariums an den Schnittstellenanschluss kann die Absperrfunktion aufgehoben werden; das Vakuum kann dann aus dem Evakuierungswegesystem in das Saugringinstrumentarium übertreten. Es versteht sich, dass eine abhängig vom Anschließen des Saugringinstrumentariums an den Schnittstellenanschluss schaltende Absperrstelle auch fern des Schnittstellenanschlusses in einem weiter innen liegenden Teil des Evakuierungswegesystems liegen kann.

Der Testbetriebslauf beinhaltet die Einstellung einer maximalen Pumpleistung mindestens einer Vakuumpumpe der Vakuumpumpanordnung. Dies gestattet es, den an einem gegebenen Einsatzort zu einer gegebenen Zeit möglichen maximalen Arbeitsbereich der Vakuumpumpanordnung zu ermitteln, d.h. den bestmöglich erzielbaren relativen Unterdruck.

Sofern die Vakuumpumpanordnung zwei (oder mehr) getrennt betreibbare Vakuumpumpen umfasst, kann die Steuereinheit dazu eingerichtet sein, während des Testbetriebslaufs die beiden Vakuumpumpen nacheinander zu betreiben und für jede der beiden Pumpen einen Wert für den Differenzdruck zu ermitteln. Die beiden Pumpen können beispielsweise als Haupt- und Hilfspumpe organisiert sein, wobei die Hilfspumpe dann in Arbeit tritt, wenn die Hauptpumpe ausfällt. Da die Anforderungen an die Saugkraft des Saugrings auch nach Hinzutreten der Hilfspumpe erfüllt werden sollen, ist es zweckmäßig, im Rahmen des Testbetriebslaufs zuerst die eine der Pumpen mit maximaler Pumpleistung zu betreiben, diese Pumpe sodann herunterzufahren und anschließend die andere Pumpe ebenfalls mit maximaler Pumpleistung zu betreiben. Dies gestattet es, etwaige Leistungsunterschiede und dementsprechend Unterschiede im Arbeitsbereich der beiden Pumpen zu detektieren.

In einer bevorzugten Ausgestaltung der Erfindung ist die Steuereinheit dazu eingerichtet, den gemessenen Vakuumdruck oder/und eine abhängig von dem gemessenen Vakuumdruck ermittelte Größe mit mindestens einem vorgegebenen Schwellenwert zu vergleichen und abhängig vom Ergebnis des Vergleichs eine vorbestimmte Reaktion zu bewirken. Die vorbestimmte Reaktion kann beispielsweise eine Betriebssperrung mindestens einer Komponente der augenchirurgischen Einrichtung umfassen, falls das Vergleichsergebnis ein unzureichendes Vakuum anzeigt. Die zu sperrende Komponente kann beispielsweise eine motorische Antriebseinheit für einen mit einer Schneidklinge bestückten mechanischen Schneidkopf eines Mikrokeratoms sein. Alternativ kann sie beispielsweise eine Laserquelle sein, die bei Feststellung eines unzureichenden erzielbaren Vakuums von der Steuereinheit gegen die Abgabe von Laserstrahlung gesperrt wird.

Die vorbestimmte Reaktion kann alternativ oder ergänzend die Ausgabe eines optisch oder/und akustisch wahrnehmbaren Warnhinweises umfassen, falls das Vergleichsergebnis ein unzureichendes Vakuum anzeigt. Der Warnhinweis kann beispielsweise in Form einer Textmeldung auf einem Monitor ausgegeben werden oder er kann darin bestehen, dass eine Signallampe gemäß einem vorbestimmten Muster (z.B. Blinken oder Dauerleuchten) aktiviert wird.

Vorzugsweise ist die Steuereinheit dazu eingerichtet, den Differenzdruck oder/und einen gegenüber dem Differenzdruck um ein vorgegebenes Maß verminderten Druckwert mit mindestens einem vorgegebenen Schwellenwert zu vergleichen. Indem ein gegenüber dem Differenzdruck verminderter Druckwert zur Grundlage des Vergleichs gemacht wird, besteht ein gewisses Sicherheitsband, innerhalb dessen das maximal erzielbare Vakuum schwanken kann, ohne als nicht mehr ausreichend qualifiziert zu werden. Das vorgegebene Maß kann beispielsweise ein prozentualer Abschlag sein; denkbar ist auch ein absoluter Abschlag.

Es hat sich gezeigt, dass in vielen Fällen ein relativer Unterdruck zwischen -350 mmHg und -650 mmHg, vorzugsweise zwischen -550 mmHg und -600 mmHg als notwendig und wünschenswert für ein zuverlässiges Anhaften des Saugrings am Auge angesehen wird. Dementsprechend ist es bevorzugt, einen Schwellenwert in diesem Bereich festzulegen. Eine herstellerseitige Festlegung ohne Änderungsmöglichkeit durch den Benutzer ist dabei denkbar. Alternativ oder zusätzlich kann mindestens ein Schwellenwert benutzerseitig einstellbar sein.

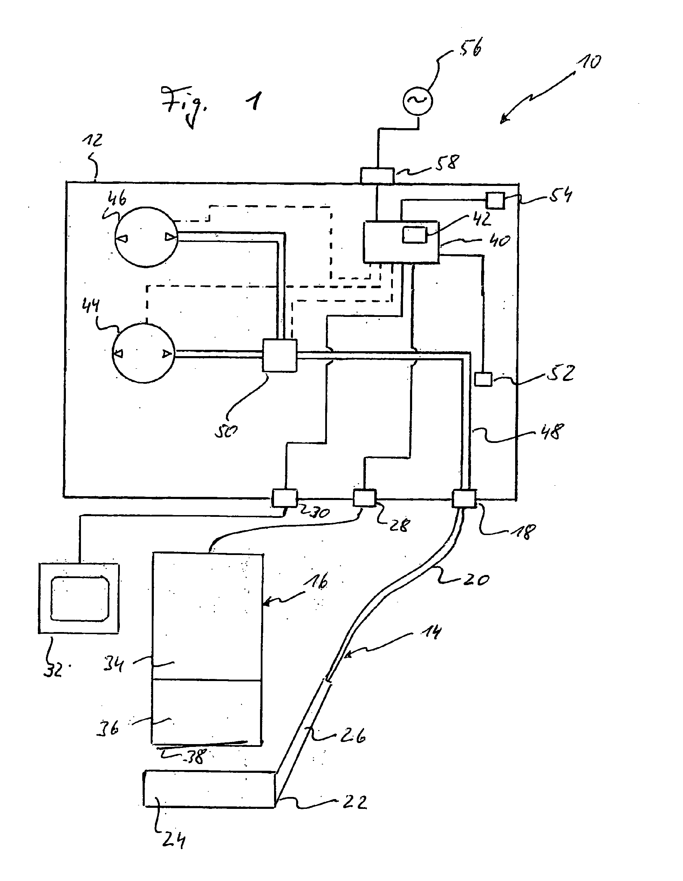

Die Erfindung wird nachfolgend anhand der beigefügten Zeichnung weiter erläutert. Deren Fig. 1 stellt schematisch ein Ausführungsbeispiel einer augenchirurgischen Einrichtung 10 dar, die im gezeigten Beispielfall als Mikrokeratom ausgebildet ist. Das Mikrokeratom dient zur Erzeugung eines kornealen Flaps an einem nicht näher dargestellten humanen Auge im Rahmen einer LASIK- oder Epi-LASIK-Operation. Das Mikrokeratom 10 umfasst eine zweckmäßigerweise als Tischgerät ausgestaltete Steuerkonsole 12, ein Saugringinstrumentarium 14 sowie ein Schneid-Handstück 16. Zum Anschluss des Saugringinstrumentariums 14 an die Steuerkonsole 12 weist diese einen Vakuum-Schnittstellenanschluss 18 auf, an den eine Schlauchleitung 20 des Saugringinstrumentariums 14 lösbar anschließbar ist. Das Saugringinstrumentarium 14 weist des weiteren eine beispielsweise einstückig aus einem Leichtmetall, z.B. Titan, hergestellte Saugringeinheit 22 mit einem Saugring 24 sowie einem von diesem abstehenden Anschlussstutzen 26 auf, mit dem die Schlauchleitung 20 vorzugsweise lösbar verbunden ist.

Elektrische Schnittstellenanschlüsse 28, 30 erlauben darüber hinaus den Anschluss des Schneid-Handstücks 16 sowie eines externen Monitors 32 an die Steuerkonsole 12. Gewünschtenfalls kann die Steuerkonsole 12 alternativ oder zusätzlich zu dem externen Monitor 32 eine integrierte Anzeigeeinheit (nicht näher dargestellt) zur Anzeige von Informationen aufweisen.

Das Schneid-Handstück 16 ist im gezeigten Beispielfall modular aufgebaut und setzt sich aus einem Antriebsmodul 34 und einem lösbar mit diesem gekoppelten Schneidmodul 36 zusammen. Das Schneidmodul 36 ist mit einer auswechselbaren Schneidklinge 38 bestückt, deren Schneidkante um ein die Dicke des zu erzeugenden Flaps bestimmendes Maß gegenüber einer nicht näher bezeichneten, eine Einebnung der Kornea bewirkenden Applanationsfläche des Schneidmoduls 36 übersteht. Das Antriebsmodul 34 ist mit motorischen Antriebskomponenten ausgestattet, um zum einen die Schneidklinge 38 innerhalb des Schneidmoduls 36 in hochfrequente laterale Oszillation zu versetzen und zum anderen das Schneid-Handstück 16 als Ganzes in einer linearen Richtung gegenüber dem Saugring 24 vor- und zurückzubewegen. Beispielsweise kann die Saugringeinheit 22 gezahnte Führungsbahnen aufweisen, in welche an dem Schneidmodul 36 angeordnete, durch das Antriebsmodul 34 angetriebene Ritzel kämmend eingreifen.

Die Steuerkonsole 12 enthält eine den gesamten Betrieb des Mikrokeratoms 10 steuernde, prozessorbasierte Steuereinheit 40, welche nach Maßgabe eines Steuerprogramms 42 arbeitet. Enthalten in der Steuerkonsole 12 ist zudem eine im gezeigten Beispielfall aus zwei Vakuumpumpen 44, 46 bestehende Vakuumpumpanordnung, welche der Erzeugung eines über ein internes Evakuierungswegesystem 48 zu dem Vakuum-Schnittstellenanschluss 18 und von dort weiter zu dem Saugring 24 zu leitenden Vakuums dient. Die Vakuumpumpen 44, 46 sind im vorliegendem Ausführungsbeispiel als Haupt- und Zusatzpumpe organisiert, wobei eine der Pumpen, etwa die Pumpe 44, als Hauptpumpe dient und die andere, etwa die Pumpe 46, nur bei Bedarf zugeschaltet wird, beispielsweise wenn die Hauptpumpe 44 ausfällt. Ein Umschalter 50 erlaubt die wahlweise Anbindung der Hauptpumpe 44 oder der Zusatzpumpe 46 an das Evakuierungswegesystem 48. Die beiden Pumpen 44, 46 sowie der Umschalter 50 werden von der Steuereinheit 40 gesteuert; dies ist durch gestrichelte Verbindungslinien in Figur 1 angedeutet. In einer Abwandlung ist es vorstellbar, auf den Umschalter 50 zu verzichten, so dass die beiden Pumpen 44, 46 permanent mit dem Evakuierungswegesystem 48 verbunden sind. Bei individueller Steuerbarkeit jeder der beiden Pumpen 44, 46 kann auch in diesem Fall eine der Pumpen in Betrieb genommen werden und die andere Pumpe betriebslos bleiben, jedenfalls so lange, wie die erste Pumpe ordnungsgemäß funktioniert.

Teil der Steuerkonsole 12 sind ferner zwei Drucksensoren 52, 54, welche ihre Sensorsignale an die Steuereinheit 40 liefern, die daraus Druckmesswerte ermittelt. Der Drucksensor 52 ist dem Evakuierungswegesystem 48 zugeordnet und misst den Vakuumdruck (d.h. den absoluten Druck) in dem Evakuierungswegesystem 48. Der Drucksensor 54 dient dagegen zur Messung des Umgebungsluftdrucks (Atmosphärendruck). Die Druckmesswerte der beiden Sensoren 52, 54 werden von der Steuereinheit 40 einer Differenzbildung unterzogen, um den relativen Unterdruck in dem Evakuierungswegesystem 48 zu ermitteln, d.h. die Differenz zwischen dem Umgebungsluftdruck und dem erzeugten Vakuumdruck in dem Evakuierungswegesystem 48.

Bei 56 ist eine elektrische Netzversorgung angedeutet, aus welcher das Mikrokeratom 10 seine für den Betrieb benötigte elektrische Energie bezieht. Ein außen an der Steuerkonsole 12 angeordneter Hauptschalter 58 erlaubt das Ein- und Ausschalten der Steuerkonsole 12 und damit des Mikrokeratoms 10 insgesamt.

Der Vakuum-Schnittstellenanschluss 18 ist in nicht näher dargestellter Weise mit einem geeigneten Absperrorgan (z.B. Sperrklappe) ausgeführt, welches das Evakuierungswegesystem 48 nach außen dicht verschließt, wenn die Schlauchleitung 20 des Saugringinstrumentariums 14 nicht angeschlossen ist, d.h. wenn der Schnittstellenanschluss 18 unbelegt ist. Durch das Anstecken der Schlauchleitung 20 wird das Absperrorgan geöffnet, so dass der Durchgang von dem Evakuierungswegesystem 48 über den Schnittstellenanschluss 18 in die Schlauchleitung 20 offen ist.

Das Steuerprogramm 42 enthält Instruktionen, die einen Testbetriebslauf der Steuerkonsole 12 nach jedem Einschalten des Hauptschalters 58 vorsehen. Im Rahmen des Testbetriebslaufs wird jede der beiden Pumpen 44, 46 nacheinander angefahren und mit maximaler Pumpleistung betrieben. Sofern dem Evakuierungswegesystem 48 weitere steuerbare Komponenten zugeordnet sind, mittels dessen die Stärke des Vakuums in dem Evakuierungswegesystem 48 beeinflussbar ist, werden auch diese Komponenten im Rahmen des Testbetriebslaufs so eingestellt, dass das in dem Evakuierungswegesystem 48 herrschende Vakuum maximal ist. Ein Beispiel einer solchen Komponente kann eine Fremdlufteinleitungsstelle mit justierbarem Fremdlufteinleitungsquerschnitt sein, wie sie in EP 1 743 609 A1 beschrieben ist.

Die Pumpen 44, 46 werden im Rahmen des Testbetriebslaufs für eine ausreichende Zeitdauer mit maximaler Pumpleistung betrieben. Während des Maximalbetriebs erfasst die Steuereinheit 40 mittels des Drucksensors 52 den Vakuumdruck in dem Evakuierungswegesystem 48; dieser stellt somit unter den gegebenen Bedingungen (Höhenlage des Einsatzorts des Mikrokeratoms 10, aktuelle Wettersituation) den bestmöglich erreichbaren Vakuumdruck dar. Unter Hinzuziehung des von dem Drucksensor 54 gemessenen Umgebungsluftdrucks ermittelt die Steuereinheit 40 sodann den Differenzdruck zwischen dem Umgebungsluftdruck und dem Vakuumdruck und vergleicht diesen Differenzdruck mit einer vorgegebenen, permanent gespeicherten oder benutzerseitig einstellbaren Schwelle. Der ermittelte Differenzdruck repräsentiert den unter den gegebenen Bedingungen bestmöglich erzielbaren relativen Unterdruck. Der Vergleich mit der gegebenen Schwelle erlaubt es der Steuereinheit 40 festzustellen, ob der relative Unterdruck ausreichend ist, um eine Operation mit dem Mikrokeratom 10 ohne Gefahr einer Saugringablösung von dem Auge durchführen zu können.

Der obige Vergleich wird getrennt für jede der Pumpen 44, 46 durchgeführt, so dass die Steuereinheit 40 unabhängig für beide Pumpen deren maximalen Arbeitsbereich unter den gegebenen Bedingungen ermitteln kann. Vorzugsweise findet der Testbetriebslauf nur statt und ist nur auslösbar, wenn der Vakuum-Schnittstellenanschluss 18 unbelegt ist, also das erwähnte Absperrorgan geschlossen ist. Je nach Ergebnis des Testbetriebslaufs kann die Steuereinheit 40 unterschiedliche Reaktionen bewirken. Falls der erzielbare relative Unterdruck nicht ausreichend ist, ist es vorstellbar, dass die Steuereinheit 40 die elektrische Energiezufuhr zu dem Schneid-Handstück 16 über den elektrischen Schnittstellenanschluss 28 sperrt und damit das Schneid-Handstück 16 betriebsunfähig macht. Alternativ oder zusätzlich kann die Steuereinheit 40 eine geeignete Warnmeldung auf dem Monitor 32 ausgeben. Die Wammeldung kann Angaben über den erzielbaren relativen Unterdruck enthalten, damit der Operateur genau weiß, welche Druckwerte er erwarten kann.

Zweckmäßigerweise wird vor dem Vergleich des gemessenen Differenzdrucks mit der Schwelle ein prozentualer oder absoluter Abschlag von dem Differenzdruck abgezogen. Dieser Abschlag kann dazu dienen, etwaigen Schwankungen der maximalen Pumpleistung der Pumpen 44, 46 sowie etwaigen Messungenauigkeiten Rechnung zu tragen.

Die bei dem Vergleich betrachtete Schwelle kann beispielsweise auf -570 mmHg eingestellt oder einstellbar sein. Es versteht sich, dass andere Zahlenwerte genauso für die Schwelle denkbar sind. Zweckmäßigerweise ist die Schwelle jedoch in einem Bereich zwischen -550 mmHg und -600 mmHg eingestellt oder einstellbar. Dies entspricht einem relativen Unterdruck, wie er für Augenoperationen vielfach als notwendig und ausreichend angesehen wird.

## Patentansprüche

1. Einrichtung für die Augenchirurgie, umfassend
- eine Vakuumpumpanordnung (44, 46) zur Erzeugung eines zur Fixierung eines Saugrings (24) an einem Auge dienenden Vakuums,
- ein Evakuierungswegesystem (48) zur Übertragung des Vakuums zu einem Schnittstellenanschluss (18), welcher den lösbaren Anschluss eines den Saugring umfassenden Saugringinstrumentariums (14) gestattet,
- eine Steuereinheit (40) zur Steuerung der Vakuumpumpanordnung,
- Druckmesskomponenten (52, 54) zur Messung zumindest des Vakuumdrucks, **dadurch gekennzeichnet, dass** die Steuereinheit dazu eingerichtet ist, einen Differenzdruck zwischen dem gemessenen Vakuumdruck und einem Atmosphärendruck zu ermitteln,
und dadurch, dass die Steuereinheit (40) dazu eingerichtet ist, einen Testbetriebslauf mindestens einer Vakuumpumpe der Vakuumpumpanordnung (44, 46) mit einer vorbestimmten Einstellung der Pumpleistung zu bewirken und den Differenzdruck auf Grundlage eines oder mehrerer während dieses Testbetriebslaufs gemessener Werte für den Vakuumdruck zu ermitteln, wobei der Testbetriebslauf die Einstellung einer maximalen Pumpleistung mindestens einer Vakuumpumpe der Vakuumpumpanordnung (44, 46) umfasst.

2. Einrichtung nach Anspruch 1, wobei die Druckmesskomponenten (52, 54) auch zur Messung des Atmosphärendrucks ausgebildet sind und die Steuereinheit (40) dazu eingerichtet ist, den Differenzdruck aus dem gemessenen Vakuumdruck und dem gemessenen Atmosphärendruck zu ermitteln.

3. Einrichtung nach Anspruch 1, wobei die Steuereinheit (40) dazu eingerichtet ist, den Testbetriebslauf nach jedem Einschalten einer elektrischen Energieversorgung (56) der Steuereinheit (40) und der Vakuumpumpanordnung (44, 46) durchzuführen.

4. Einrichtung nach einem der vorhergehenden Ansprüche, wobei die Steuereinheit (40) dazu eingerichtet ist, den Testbetriebslauf ohne an den Schnittstellenanschluss (18) angeschlossenes Saugringinstrumentarium (14) durchzuführen.

5. Einrichtung nach Anspruch 4, wobei die Steuereinheit (40) dazu eingerichtet ist, den Testbetriebslauf nur dann durchzuführen, wenn das Saugringinstrumentarium (14) nicht an den Schnittstellenanschluss (18) angeschlossen ist.

6. Einrichtung nach einem der vorhergehenden Ansprüche , wobei die Vakuumpumpanordnung (44, 46) zwei getrennt betreibbare Vakuumpumpen umfasst und die Steuereinheit (40) dazu eingerichtet ist, während des Testbetriebslaufs die beiden Vakuumpumpen nacheinander zu betreiben und für jede der beiden Pumpen einen Wert für den Differenzdruck zu ermitteln.

7. Einrichtung nach einem der Ansprüche 1 bis 6, wobei die Steuereinheit (40) dazu eingerichtet ist, den gemessenen Vakuumdruck oder/und eine abhängig von dem gemessenen Vakuumdruck ermittelte Größe mit mindestens einem vorgegebenen Schwellenwert zu vergleichen und abhängig vom Ergebnis des Vergleichs eine vorbestimmte Reaktion zu bewirken.

8. Einrichtung nach Anspruch 7, wobei die Steuereinheit (40) dazu eingerichtet ist, den Differenzdruck oder/und einen gegenüber dem Differenzdruck um ein vorgegebenes Maß verminderten Druckwert mit mindestens einem vorgegebenen Schwellenwert zu vergleichen.

9. Einrichtung nach Anspruch 8, wobei ein Schwellenwert zwischen -350 mmHg und -650 mmHg, vorzugsweise zwischen -550 mmHg und -600 mmHg liegt.

10. Einrichtung nach Anspruch 8 oder 9, wobei mindestens ein Schwellenwert benutzerseitig einstellbar ist.

11. Einrichtung nach einem der Ansprüche 7 bis 10, wobei die vorbestimmte Reaktion eine Betriebssperrung mindestens einer Komponente (16) der augenchirurgischen Einrichtung umfasst, falls das Vergleichsergebnis ein unzureichendes Vakuum anzeigt.

12. Einrichtung nach Anspruch 11, wobei die Betriebssperrung zumindest eine motorische Antriebseinheit (34) für eine mit einer Schneidklinge (38) bestückte mechanische Schneideinheit (16) eines Mikrokeratoms betrifft.

13. Einrichtung nach einem der Ansprüche 7 bis 12, wobei die vorbestimmte Reaktion die Ausgabe eines optisch oder/und akustisch wahrnehmbaren Warnhinweises umfasst, falls das Vergleichsergebnis ein unzureichendes Vakuum anzeigt.

## Claims

1. A device for ophthalmic surgery, comprising:
- a vacuum pump arrangement (44, 46) for generating a vacuum serving for fixing a suction ring (24) to an eye,
- an evacuation path system (48) for transmitting the vacuum to an interface port (18) which permits the separable attachment of the suction ring instrumentarium (14) including the suction ring,
- a control unit (40) for controlling the vacuum pump arrangement,
- pressure measuring components (52, 54) for measuring at least the vacuum pressure,
**characterised in that** the control unit (40) is adapted to determine a differential pressure between the measured vacuum pressure and an atmospheric pressure, and **in that** the control unit (40) is adapted to bring about a test operation run of at least one vacuum pump of the vacuum pump arrangement (44, 46) with a predetermined setting of the pumping power and to determine the differential pressure on the basis of one or several values of the vacuum pressure measured during this test operation run, wherein the test operation run includes the setting of a maximum pumping power of at least one vacuum pump of the vacuum pump arrangement (44, 46).

2. The device according to Claim 1, wherein the pressure measuring components (52, 54) are also designed for measuring the atmospheric pressure and the control unit (40) is adapted to determine the differential pressure from the measured vacuum pressure and the measured atmospheric pressure.

3. The device according to Claim 1, wherein the control unit (40) is adapted to bring about the test operation run after each switching-on of an electrical power supply (56) of the control unit (40) and the vacuum pump arrangement (44, 46).

4. The device according to one of the previous claims, wherein the control unit (40) is adapted to bring about the test operation run without the suction ring instrumentarium (14) being connected to the interface port (18).

5. The device according to Claim 4, wherein the control unit (40) is adapted to bring about the test operation run only, if the suction ring instrumentarium (14) is not connected to the interface port (18).

6. The device according to one of the previous claims, wherein the vacuum pump arrangement (44, 46) comprises two separately operable vacuum pumps and the control unit (40) is adapted to operate the two vacuum pumps successively during the test operation run and to determine a value of the differential pressure for each of the two pumps.

7. The device according to one of Claims 1 to 6, wherein the control unit (40) is adapted to compare the measured vacuum pressure and/or a quantity which is determined dependent on the measured vacuum pressure with at least one predetermined threshold value and to bring about a predetermined response depending on the result of the comparison.

8. The device according to Claim 7, wherein the control unit (40) is adapted to compare the differential pressure and/or a pressure value reduced by a predetermined amount in relation to the differential pressure with at least one predetermined threshold value.

9. The device according to Claim 8, wherein a threshold value lies between -350 mmHg and -650 mmHg, preferably between 550 mmHg and -600 mmHg.

10. The device according to Claim 8 or 9, wherein at least one threshold value may be set by a user.

11. The device according to one of Claims 7 to 10, wherein the predetermined response includes an operational disabling of at least one component (16) of the ophthalmic surgery device if the comparison result indicates an insufficient vacuum.

12. The device according to Claim 11, wherein the operational disabling relates to at least one motorised drive unit (34) for a mechanical cutting unit (16) of a micro-keratome equipped with a cutting blade (38).

13. The device according to one of Claims 7 to 10, wherein the predetermined response includes the output of a visually and/or acoustically perceptible warning indication the comparison result indicates an insufficient vacuum.

## Revendications

1. Dispositif pour la chirurgie oculaire, comprenant
- un ensemble pompe à vide (44, 46) pour générer un vide servant à la fixation d'un anneau de succion (24) sur un oeil,
- un système de voies d'évacuation (48) pour transmettre le vide à un raccord d'interface (18) qui permet le raccordement détachable d'un instrument à anneau de succion (14) comprenant l'anneau de succion,
- une unité de commande (40) pour commander l'ensemble pompe à vide,
- des composants de mesure de pression (52, 54) pour mesurer au moins la pression de vide,
**caractérisé en ce que** l'unité de commande est conçue pour déterminer une pression différentielle entre la pression de vide mesurée et une pression atmosphérique,
et **en ce que** l'unité de commande (40) est conçue pour produire un cycle de fonctionnement d'essai d'au moins une pompe à vide de l'ensemble pompe à vide (44, 46) avec un réglage prédéterminé de la puissance de pompage et pour déterminer la pression différentielle sur la base d'une ou plusieurs valeurs mesurées pour la pression de vide pendant ce cycle de fonctionnement d'essai, le cycle de fonctionnement d'essai comprenant le réglage d'une puissance de pompage maximale d'au moins une pompe à vide de l'ensemble pompe à vide (44, 46).

2. Dispositif selon la revendication 1, dans lequel les composants de mesure de pression (52, 54) sont aussi conçus pour mesurer la pression atmosphérique, et l'unité de commande (40) est conçue pour déterminer la pression différentielle à partir de la pression de vide mesurée et de la pression atmosphérique mesurée.

3. Dispositif selon la revendication 1, dans lequel l'unité de commande (40) est conçue pour exécuter le cycle de fonctionnement d'essai après chaque mise sous tension d'une alimentation en énergie électrique (56) de l'unité de commande (40) et de l'ensemble pompe à vide (44, 46).

4. Dispositif selon l'une des revendications précédentes, dans lequel l'unité de commande (40) est conçue pour exécuter le cycle de fonctionnement d'essai sans instrument à anneau de succion (14) raccordé au raccord d'interface (18).

5. Dispositif selon la revendication 4, dans lequel l'unité de commande (40) est conçue pour exécuter le cycle de fonctionnement d'essai seulement lorsque l'instrument à anneau de succion (14) n'est pas raccordé au raccord d'interface (18).

6. Dispositif selon l'une des revendications précédentes, dans lequel l'ensemble pompe à vide (44, 46) comprend deux pompes à vide pouvant fonctionner séparément, et l'unité de commande (40) est conçue pour faire fonctionner les deux pompes à vide l'une après l'autre pendant le cycle de fonctionnement d'essai et pour déterminer une valeur de la pression différentielle pour chacune des deux pompes.

7. Dispositif selon l'une des revendications 1 à 6, dans lequel l'unité de commande (40) est conçue pour comparer la pression de vide mesurée et/ou une grandeur déterminée en fonction de la pression de vide mesurée avec au moins une valeur seuil prédéfinie et pour produire une réaction prédéterminée en fonction du résultat de la comparaison.

8. Dispositif selon la revendication 7, dans lequel l'unité de commande (40) est conçue pour comparer la pression différentielle et/ou une valeur de pression diminuée d'une quantité prédéfinie avec au moins une valeur seuil prédéfinie.

9. Dispositif selon la revendication 8, dans lequel une valeur seuil se situe entre -350 mmHg et -650 mmHg, de préférence entre -550 mmHg et -600 mmHg.

10. Dispositif selon la revendication 8 ou 9, dans lequel au moins une valeur seuil est réglable par l'utilisateur.

11. Dispositif selon l'une des revendications 7 à 10, dans lequel la réaction prédéterminée comprend un blocage de fonctionnement d'au moins un composant (16) du dispositif de chirurgie oculaire si le résultat de la comparaison indique un vide insuffisant.

12. Dispositif selon la revendication 11, dans lequel le blocage de fonctionnement concerne au moins une unité (34) d'entraînement motorisée pour une unité (16) de coupe mécanique équipée d'une lame de coupe (38) d'un microkératome.

13. Dispositif selon l'une des revendications 7 à 12, dans lequel la réaction prédéterminée comprend l'émission d'un signal d'avertissement optique et/ou acoustique si le résultat de la comparaison indique un vide insuffisant.
